# FASCICULE DE BREVET EUROPEEN

(11) **EP 0 691 620 B1**
(45) Date de publication et mention de la délivrance du brevet: **02.03.2005**
(21) Numéro de dépôt: 95401584.8
(22) Date de dépôt: 30.06.1995
(51) Int. Cl.: G06F 19/00

(54) **Procédé de compression de données physiologiques, notamment d'activité cardiaque, en particulier pour un enregistrement Holter d'électrocardiogrammes ou d'electrogrammes**
Verfahren zur Komprimierung physiologischer Daten, insbesondere der Herzaktivität, insbesondere für Holteraufzeichnung von Elektrokardiogrammen oder Electrogrammen
Compression procedure of physiological data, expecially of cardiac activity, in particular for Holter type of recording of electrocardigrams or electrograms

(30) Priorité: 07.07.1994 FR 9408406
(43) Date de publication de la demande: 10.01.1996
(73) Titulaire: ELA MEDICAL (Société anonyme), 92541 Montrouge (FR)
(72) Inventeur: Faisandier, Yves, F-75014 Paris (FR)
(74) Mandataire: Dupuis-Latour, Dominique

(56) Documents cités:
- EP-A- 0 129 981
- EP-A- 0 552 009
- MEDICAL AND BIOLOGICAL ENGINEERING AND COMPUTING, vol.29, no.2, Mars 1991, STEVENAGE GB pages 175 - 179, XP000230957 S.C.TAI 'SLOPE - A REAL TIME ECG DATA COMPRESSOR'
- ELECTRONIC ENGINEERING, vol.63, no.780, Décembre 1991, LONDON GB page 30, XP000256938 LIN JUN 'FULL USE OF MEMORY FOR 12BIT ADC DATA ACQUISITION'
- PROCEEDINGS OF COMPUTERS IN CARDIOLOGY, IEEE PRESS NEW YORK US, 23 Septembre 1991, VENEZIA, IT pages 593 - 596, XP000312491 G.PASSARIELLO ET AL 'ARITHMETIC CODING FOR ECG DATA COMPRESSION'
- PROCEEDINGS OF COMPUTERS IN CARDIOLOGY, IEEE PRESS NEW YORK US, 25 Septembre 1988, WASHINGTON US pages 465 - 468, XP000145693 PENG WIE HSIN 'ELECTROCARDIOGRAPHIC DATA COMPRESSION USING PRECEDING CONSECUTIVE QRS INFORMATION'
- PROCEEDINGS OF COMPUTERS IN CARDIOLOGY, IEEE PRESS NEW YORK US, 25 Septembre 1988, WASHINGTON US pages 575 - 578, XP000145718 P.J.NARRAMORE ET AL 'OPTIMISING SOLID STATE HOLTER SYSTEM'

## Description

L'invention concerne un procédé de compression de donnés physiologiques.

Il peut s'agir notamment de donnés relatives à une activité cardiaque, une telle compression étant particulièrement adaptée aux enregistrements dits "Holter", c'est-à-dire aux enregistrements effectués en continu et sur une longue période de signaux recueillis au moyen d'électrodes implantées ("électrogrammes") ou d'électrodes externes ("électrocardiogrammes").

Ce type d'enregistrement peut également concerner d'autres données physiologiques telles que rythme respiratoire, tension artérielle, etc. et l'on comprendra que la présente invention est directement applicable à la compression de telles autres données. Aussi, bien que, dans la suite de la description, on se référera principalement à la compression d'enregistrements Holter d'électrocardiogrammes (ECG), qui constitue l'application la plus fréquente, cet aspect n'est en aucune façon limitatif.

L'invention s'applique en effet parfaitement à la compression de données produites par des appareils implantés tels que (dans le cas de données d'activité cardiaque) stimulateurs, cardioverteurs ou défibrillateurs implantés. En outre, il peut s'agir tout aussi bien de la compression de données, lors de leur acquisition, en vue de leur enregistrement dans l'un de ces appareils implantés que de la transmission en différé vers l'extérieur d'informations déjà enregistrées dans l'appareil implanté. Dans ce dernier cas, il peut en effet être souhaitable de comprimer les données à transmettre pour limiter le temps de transmission des informations vers le programmateur externe compte tenu du faible débit du flux d'informations, limité par la bande passante relativement étroite des systèmes de transmission utilisables dans ce contexte.

Ces enregistrements Holter, autrefois réalisés sur bande magnétique compte tenu du volume important d'informations à mémoriser, sont aujourd'hui effectués de plus en plus souvent dans une mémoire statique à semiconducteurs, dite "mémoire Holter". L'utilisation de cette technologie est cependant limitée par la capacité moindre des mémoires statiques par rapport aux bandes magnétiques, ce qui oblige, au moment de l'acquisition des données et avant leur mémorisation, à utiliser un algorithme de compression pour économiser la place disponible dans la mémoire Holter.

Ainsi, si l'on veut enregistrer pendant 24 heures les signaux recueillis sur deux voies ECG distinctes, et que le signal ECG de chaque voie est échantillonné à 100 Hz avec une résolution de 10 µV et une dynamique de 10 mV, le flux d'informations sur chaque voie est de 1000 bits/seconde (100 mots de 10 bits chacun). Si, par exemple, on dispose de mémoires statiques permettant une capacité de mémoire Holter de 10 Mo, sur laquelle on réserve 1 Mo pour les fichiers de résultats d'analyses, d'histogrammes, etc., il reste 4,5 Mo disponibles pour mémoriser les 24 heures de signaux de chacune des voies. Cette contrainte de taille impose une limitation du flux de données à mémoriser à 52,08 octets/seconde soit 422,4 bits/seconde ; il est donc nécessaire de comprimer les données avec un rapport égal à au moins 1000 / 422,4 = 2,36.

Pour un signal plus riche en informations, le processeur peut échantillonner l'ECG à une fréquence plus élevée, de 200 Hz par exemple. Toutes choses égales par ailleurs, le taux de compression doit alors passer à 4,72.

Beaucoup de procédés ont été proposés pour comprimer ces données. Le problème particulier posé par l'ECG enregistré en ambulatoire est sa non-régularité et la présence de nombreux artéfacts. En effet, le signal ECG est constitué le plus souvent du signal d'origine cardiaque, presque périodique (complexe PQRST), accompagné de signaux générés par les muscles, par les perturbations mécaniques de l'interface électrode-peau, ou de perturbations électriques ou encore électromagnétiques reçues par les câbles reliant les électrodes à l'enregistreur.

Les algorithmes classiques se révèlent relativement peut performants dans le cas particulier des signaux ECG. D'une part, s'agissant de signaux qui sont toujours parasités de façon relativement importante, les algorithmes classiques savent assez mal éliminer ces parasites.

D'autre part, il est important que la compression/décompression ne produise pas en elle-même d'artéfacts trop visibles à l'oeil nu, comme c'est par exemple le cas des compressions utilisant des approximations par arcs de parabole ou segments de droite, qui introduisent à l'écran des "cassures" très visibles à l'oeil et susceptibles de gêner l'interprétation du tracé ECG par le praticien, en donnant à ce tracé une allure éloignée de ceux, obtenus directement, qu'il a l'habitude d'observer et d'interpréter.

Enfin, toujours pour un signal ECG, il est important de pouvoir observer la variabilité du complexe QRS, qui peut être très significative pour le diagnostic, et donc pouvoir conserver un certain nombre de micro-variations.

Cette contrainte rend, en pratique, peu efficace les algorithmes de compression prédictifs basés sur la répétition d'un signal moyen (le complexe PQRST), car la multitude de formes de PQRST différents rend leur travail très difficile et leur performance se dégrade rapidement. L'algorithme de compression ne doit pas seulement fonctionner dans le cas idéal, c'est-à-dire pour un ECG régulier et sans parasites, mais doit pouvoir supporter des ECG très irréguliers (complexes anormaux fréquents, de forme très variable par exemple, avec des périodes stables), contenant souvent des artéfacts.

Une autre difficulté typique des enregistrements Holter est le fait que les appareils doivent pouvoir fonctionner sur piles avec une autonomie devant pouvoir atteindre 24 heures, parfois même 48 heures. Or l'utilisation d'algorithmes complexes nécessite un processeur suffisamment puissant fonctionnant de façon quasi-permanente, donc impliquant une forte consommation énergétique et une limitation de la durée d'enregistrement, à moins d'augmenter de façon excessive le poids et l'encombrement de l'appareil.

L'un des buts de l'invention est donc de proposer un nouveau procédé de compression de données physiologiques particulièrement bien adapté aux contraintes des enregistrements Holter, parmi lesquelles :
- l'enregistrement d'un signal très irrégulier dans le temps,
- la présence de nombreux parasites et artéfacts, qui ne devront pas conduire à une dégradation du signal utile du fait de la compression,
- la capacité limitée des mémoires statiques actuelles, donc la nécessité de comprimer les données avec un taux de compression élevé, sans pour autant dégrader sensiblement l'information mémorisée,
- l'autonomie énergétique limitée des appareils, qui impose le choix d'un algorithme rapide afin de réduire au maximum le taux d'utilisation du processeur, donc sa consommation globale.

Pour atteindre ces buts, l'invention propose un procédé caractérisé par les étapes successives suivantes : (a) acquisition des données par recueil d'un signal électrique et échantillonnage de ce signal à une fréquence donnée ; (c) détermination de la dérivée première du signal échantillonné ; (e) détermination de la dérivée seconde de ce même signal échantillonné ; (f) analyse prédictive du signal échantillonné en fonction d'une pluralité de valeurs futures des dérivées première et seconde et sélection, pour l'échantillon courant, d'un mode de codage parmi une pluralité de modes de codage prédéfinis commutables dynamiquement, de manière à optimiser le volume global des codes créés pour le codage de ladite pluralité de valeurs futures ; et (g) compression des données par mise en oeuvre du mode de codage choisi à l'étape (f).

Très avantageusement, l'étape (g) comprend : (g1) en cas de changement de mode, la création d'un code de changement de mode, et (g2) la création d'un code de donnée représentatif de la valeur de l'échantillon courant, à partir de la valeur courante correspondante de dérivée première ou de dérivée seconde. Généralement, après l'étape (g), il est prévu une étape (h) de mémorisation séquentielle des codes créés aux étapes (g1) et (g2).

En outre, selon un certain nombre de formes de réalisation préférentielles, caractéristiques de l'invention :
- entre l'étape (a) et l'étape (c), il est prévu une étape (b) d'élimination des interférences à la fréquence du secteur par prétraitement des données échantillonnées ;
- à l'étape (c), la dérivée première est obtenue par détermination de l'incrément entre l'échantillon de signal courant acquis à l'étape (a) et une valeur recalculée correspondant à l'échantillon de signal précédent tel que comprimé à l'étape (g) puis décomprimé par mise en oeuvre d'une étape annexe, exécutée de façon concomitante, de codage inverse ;
- il est prévu en outre, entre l'étape (c) et l'étape (e), une étape (d) de réduction éventuelle, d'un facteur donné, de la résolution du signal échantillonné, ce facteur étant déterminé par comparaison entre un débit moyen courant du flux de données mémorisées à l'étape (h) et d'un débit nominal de consigne en augmentant le facteur de réduction lorsque le débit moyen est supérieur au débit nominal, et inversement.
- ladite pluralité de modes de codage peut notamment comprendre :
   . un premier mode de codage dans lequel on mémorise dans un mot de longueur variable la valeur de la dérivée seconde du signal échantillonné, en attribuant une longueur de mot différente pour chaque valeur possible, mode qui peut notamment être sélectionné lorsque la valeur absolue de la dérivée seconde du signal échantillonné est inférieure à une première limite prédéterminée ;
   . un second mode de codage dans lequel on mémorise dans un mot de longueur fixe la valeur de la dérivée seconde du signal échantillonné, mode qui peut notamment être sélectionné lorsque la valeur absolue de la dérivée seconde du signal échantillonné est supérieure à une première limite prédéterminée et inférieure à une deuxième limite prédéterminée ;
   . un troisième mode de codage dans lequel on mémorise dans un mot de longueur fixe la valeur de la dérivée première du signal échantillonné ou bien le résultat d'un transcodage logarithmique de cette valeur, mode qui peut notamment être sélectionné lorsque la valeur absolue de la dérivée seconde du signal échantillonné est supérieure à une troisième limite prédéterminée.

Dans un forme de mise en oeuvre avantageuse, on enregistre en outre à intervalles réguliers, dans une table d'états, la valeur courante de la dérivée première, le mode sélectionné courant et la position dans l'enregistrement de la donnée mémorisée courante.

L'invention couvre également un procédé de décompression de données comprimées et mémorisées selon un procédé tel qu'exposé ci-dessus, caractérisé par les étapes successives de lecture et individualisation des données mémorisées, détermination du mode de codage de la donnée lue courante et décompression de la donnée par codage inverse.

D'autres caractéristiques et avantages de l'invention apparaîtront à la lecture de la description détaillée ci-dessous d'un exemple de mise en oeuvre, donné en référence à la figure unique annexée qui représente l'organigramme général du procédé de compression selon l'invention.

La figure donne, de façon schématique, l'enchaînement des différentes étapes du procédé de compression de l'invention.

Essentiellement, dans le procédé de l'invention, on dispose d'une pluralité de mode de compression différents et on les sélectionne automatiquement et de façon adaptative en fonction de la richesse du signal recueilli. Le procédé peut également comporter, de façon avantageuse, un réglage de la résolution, ici encore automatique et adaptatif, permettant de modifier le débit des données en entrée de la mémoire en fonction des capacités de stockage du système.

### Acquisition et prétraitement des données ECG

À l'étape 1, on procède tout d'abord à l'acquisition des données, par numérisation du niveau de tension et échantillonnage. La fréquence d'échantillonnage peut être choisie en fonction de la qualité souhaitée du signal et des capacités de l'algorithme de compression, mais également en fonction de la facilité de réaliser un filtrage des interférences secteur.

Ce filtrage est réalisé à l'étape 2. Si l'on suppose que le signal ECG est parasité par une sinusoïde à 50 Hz (60 Hz aux USA), il est possible de réaliser un filtrage, très économique en temps de calcul, de ces parasites en moyennant (moyenne glissante) quatre points tous décalés de 90°, c'est-à-dire à 5 ms les uns des autres (fréquence d'échantillonnage = 4 x fréquence du secteur) : cette moyenne donne toujours une valeur résiduelle que ne contient plus de 50 Hz. Il est également possible de filtrer le 50 Hz en sommant deux points espacés d'un point: étant déphasés de 180° à cette fréquence, les deux valeurs s'annulent. La bande passante du filtre peut enfin être améliorée en donnant des coefficients optimisés aux points qui les entourent. On obtient ainsi un signal propre, débarrassé du signal secteur, procurant une qualité suffisante pour enregistrement de l'ECG.

De plus on peut prévoir de réaliser, outre le filtrage secteur, un filtrage passe-bas avec une fréquence de coupure de l'ordre de 25 à 30 Hz, éventuellement commutable à une fréquence plus élevée, 60 Hz par exemple, si l'on souhaite analyser des micro-variations, mais en acceptant alors, le cas échéant, un niveau de parasites plus élevé ; on peut même prévoir, dans le cas d'un signal pratiquement dépourvu de parasites, de supprimer tout filtrage par une commutation appropriée.

Ensuite, à l'étape 3, en fonction du débit d'enregistrement possible (donc de taille de la mémoire de stockage disponible), le procédé choisit un fonctionnement particulier :
- 420 bits/seconde : on sous-échantillonne un point toutes les 10 ms, ce point étant la moyenne de quatre points échantillonnés à 200 Hz. Si le signal d'origine est peu artéfacté et qu'il reste un excédent de mémoire, on prend la moyenne de deux points espacés d'un point (auquel on ajoute éventuellement la somme du point intermédiaire plus le point précédent multiplié par un coefficient de - 0,1 environ, pour relever la bande passante).
- 900 bits/seconde : si le signal est peu bruité, on échantillonne à 200 points par seconde. Si le signal est légèrement bruité ou qu'il n'existe pas d'excédent de mémoire, on réduit le bruit par filtrage sur deux points comme décrit précédemment.

On obtient ainsi en sortie un signal de donnée, noté D, représentatif du signal ECG après les différents traitements préalables que l'on vient d'indiquer.

L'étape 4 qui suit consiste à calculer la dérivée première, notée D', de cette donnée D. Par "dérivée première" on entend en fait la valeur de l'accroissement du signal entre deux points successifs. en effet, puisque le signal ECG ne contient pas de composante continue, le fait de ne conserver que la seule dérivée n'implique aucune perte d'information. D'autre part, l'intervalle d'échantillonnage étant constant, l'accroissement de la valeur du signal varie toujours de la même façon que le taux d'accroissement de cette même valeur, de sorte qu'il suffit, par commodité, de considérer simplement la variation incrémentale du signal d'un point d'échantillonnage au suivant.

De la même façon, on appellera "dérivée seconde", notée D", l'accroissement de la dérivée première (au sens entendu ci-dessus) d'un point d'échantillonnage au suivant.

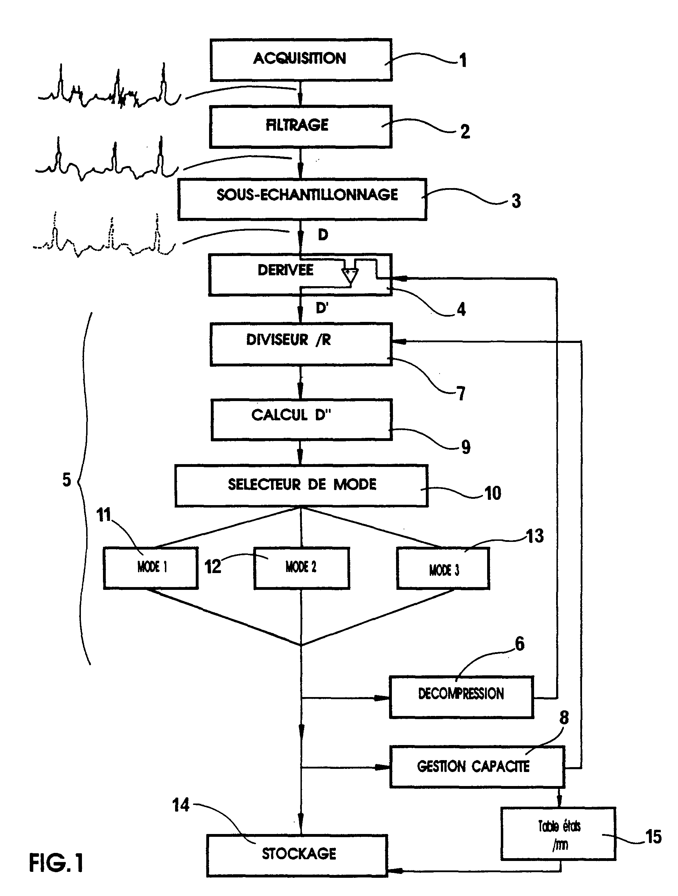

À l'étape 4, le calcul de cette dérivée première D' est effectué par détermination de la différence entre la valeur du signal au point courant et la valeur correspondante de ce même signal au point précédent. Le signal du point courant à considérer est, en tout état de cause, celui délivré après le prétraitement des étapes 1 à 3 que l'on a décrites plus haut. En ce qui concerne le point précédent, du fait que le système, dans la compression ultérieure, peut dans certains cas introduire une légère erreur dans la valeur de dérivée après compression, on préfère, à l'étape 3, déterminer la différence entre la donnée du point courant et la donnée du point précédent reconstitué, c'est-à-dire ayant subi une compression (enchaînement des diverses étapes référencées 5 sur l'organigramme de la figure 1) puis une compression inverse ou décompression (étape 6 sur la figure 1). L'erreur éventuelle n'est alors pas cumulée, car elle sera compensée à l'échantillonnage suivant. En variante, au lieu de procéder à une décompression, il est possible d'obtenir par calcul la donnée du point précédent reconstitué, en connaissant le mode de compression sélectionné et la valeur de dérivée du point précédent.

Une fois cette valeur de dérivée D' déterminée, avant de procéder à la compression proprement dite, il est avantageusement prévu une étape 7 d'ajustement adaptatif de la résolution du signal acquis.

En effet, on peut souvent tolérer qu'un signal ECG soit reproduit avec une certaine distorsion, ce qui permet bien entendu d'améliorer le score de l'algorithme de compression. Mais le risque majeur de la distorsion est de créer des défauts visibles lorsque le praticien observe le signal reconstitué. Il est en particulier indispensable, pour éviter toute erreur de lecture et de diagnostic, d'éviter que le procédé n'occulte de petites ondes ou des ondulations de faible amplitude, comme par exemple les ondes P des oreillettes, dont la pertinence est très grande pour certaines pathologies.

Si, inversement, on retient beaucoup d'informations, il ne sera pas possible de comprimer suffisamment les données et ce d'autant plus que le signal est parasité. Comme il est absolument nécessaire de limiter le débit vers la mémoire de masse, le procédé doit prévoir un moyen de réduire le débit sur les passages trop riches en informations, mais en réduisant le plus discrètement possible la qualité de l'enregistrement.

Un compromis doit donc être trouvé au cas par cas, à cet égard. Pour parvenir à ce compromis, l'invention propose d'agir de façon adaptative sur la résolution du signal acquis, opération réalisée à l'étape 7.

L'expérience a en effet montré qu'il est préférable pour des signaux ECG de faire varier la résolution plutôt que d'opérer, par exemple, un filtrage passe-bas, dans la mesure où une résolution faible sur des passages parasités se révèlent en pratique peu gênantes.

Le choix de la résolution est en fait dicté par une analyse de l'occupation de la mémoire de masse, analyse effectuée par un module de gestion de capacité (étape 8). Le critère consiste à déterminer si, compte tenu du volume d'informations déjà mémorisé depuis le début de l'acquisition, on se trouve en avance ou en retard par rapport à un débit moyen de consigne.

Cette valeur de consigne est par exemple calculée en divisant le volume d'informations (taille du fichier) stockées dans la mémoire de masse depuis le début de l'acquisition par le nombre de secondes écoulées depuis ce même moment. Ce débit réel moyen est comparé à un débit moyen de consigne, qui est dans cet exemple la taille totale de la mémoire divisée par le nombre de secondes d'un enregistrement complet, c'est-à-dire le nombre de secondes dans 24 ou 48 heures.

Si le débit réel moyen est supérieur au débit de consigne, ce qui signifie que l'on est "en avance" et que, à ce rythme, la mémoire sera totalement remplie avant la fin des 24 ou 48 heures, on réduit alors la résolution à la moitié, au tiers, au quart, etc. de la résolution de base (division par R = 2, 3, 4, etc.). Le facteur R de réduction de la résolution est choisi d'autant plus élevé que l'écart entre le débit réel moyen et le débit de consigne est important, afin de revenir plus rapidement à un débit réel proche du débit de consigne.

Inversement, si l'on est "en retard" sur la consigne, ce qui signifie que, dans les conditions courantes, la mémoire ne serait que partiellement utilisée à la fin des 24 ou 48 heures, on diminue la valeur de R afin de revenir à des données plus riches et occuper plus complètement la mémoire.

Une réduction de la résolution a pour effet une réduction immédiate du flux d'informations comprimé, du fait de la disparition des toutes petites ondes et de la réduction de la dynamique des valeurs de dérivée (le procédé comprimant d'autant plus efficacement que ces valeurs sont petites, comme on le verra ci-après). Le risque de perdre dans ce cas les petites ondes est alors sans importance, celles-ci étant le plus souvent noyées dans les artéfacts présents (un signal riche étant toujours artéfacté).

La meilleure valeur de résolution (R = 1) correspond par exemple à 10 µV, et peut être utilisée lorsque l'on se trouve dans les meilleures conditions possibles. Pour des signaux plus parasités, on peut augmenter cette valeur par paliers de 10 µV jusqu'à 40 µV (R = 4), valeur encore compatible avec des niveaux de parasites typiques, de l'ordre de 5 à 10 µV dans la majorité des situations rencontrées en pratique. Au surplus, bien que l'on ait réduit la résolution, ce chiffre de 40 µV est à comparer à la résolution typique des appareils classiques, qui est une résolution fixe de l'ordre de 50 µV. L'invention procure donc, même en "mode dégradé", c'est-à-dire avec le niveau de résolution le plus faible, une amélioration à cet égard par rapport à l'art antérieur.

On notera que l'étape 7 de réduction adaptative de la résolution est avantageusement placée après l'étape 4 de calcul de dérivée. Ainsi, un changement de résolution ne produit aucune discontinuité dans le signal, alors qu'il s'en serait produit une dans le cas inverse, dès que la donnée n'aurait pas été égale à zéro. On évite ainsi de cumuler les erreurs produites par les changements de résolution.

L'étape suivante, qui est l'étape 9, est une étape de calcul de la dérivée seconde (calcul de la différence entre la dérivée première du point courant et la dérivée première du point précédent), cette donnée étant nécessaire dans certains de modes de compression.

Le procédé passe ensuite à la compression proprement dite (étapes 10 à 13).

### Compression des données prétraitées

De façon caractéristique, le système choisit entre plusieurs modes de compression (par exemple trois modes 11, 12 et 13) en fonction de la valeur des dérivées premières D' et des dérivées secondes D" entourant chaque point considéré.

Ce principe général permet d'enregistrer sur un à quelques bits par point les passages - les plus fréquents et les plus longs d'un tracé ECG - correspondant à une dérivée seconde assez stable (segment presque plat ou pente assez régulière), et sur un nombre de bits plus important (par exemple 7 bits) les passages à forte pente ou à forte variabilité.

Le système peut basculer à tout moment d'un mode de compression à un autre en envoyant un code de changement de mode. L'algorithme de compression doit être suffisamment prédictif pour optimiser le nombre de changements de mode par seconde, ces codes venant en effet s'ajouter aux informations en réduisant le score global. En d'autres termes, connaissant à l'avance par exemple trois ou quatre valeurs de dérivée première et seconde, on évalue le score global de la compression avec ou sans changement de mode et on prend la décision de sélectionner ou non un autre mode en fonction de cette évaluation.

Dans l'exemple illustré, on va sélectionner un mode parmi trois modes 11, 12 ou 13 mais ce choix n'est pas limitatif et l'on peut sans sortir du cadre de l'invention envisager un système simplifié à deux modes, ou un système à plus de trois modes. Ces variantes seront exposées plus bas.

Dans la forme de mise en oeuvre illustrée, les trois types de codage sont les suivants :
- mode n° 1 : codage de la dérivée seconde sur un mot de longueur variable ; ce premier mode, correspondant à des faibles valeurs de dérivée, est bien adapté aux ondes lentes, qui constituent une grande partie du signal ECG.
- mode n° 2 : codage de la dérivée seconde sur un mot de longueur fixe ; ce deuxième mode est bien adapté à des valeurs moyennes de dérivée première ;
- mode n° 3 : codage direct de la dérivée première, sur un mot de longueur fixe ; ce troisième mode est bien adapté aux ondes rapides ou aux variations brutales du signal ECG (par exemple des ondes P violentes).

### Mode de codage n°1

Le codage selon le mode n° 1 (étape 11) est par exemple sélectionné tant que la valeur de dérivée seconde reste comprise entre +2 et -2 unités (soit ± 20 mV si l'unité est de 10 mV).

Le programme choisi alors de stocker la valeur de la dérivée seconde en affectant un mot de longueur variable à chaque valeur possible (principe du codage d'Huffmann). Comme, statistiquement, la valeur 0 est la plus fréquente, on attribue un mot de 1 bit (par exemple la valeur '0') à cette valeur ; les valeurs +1 et moins -1 venant ensuite assez fréquemment, on attribue la valeur '10' à +1, et la valeur '110' à -1 ; enfin, pour +2 et -2, on attribue les valeurs '1110' et '11110' respectivement.

En d'autres termes, on est en présence d'un "dictionnaire" figé de dérivées secondes, utilisé tant que la valeur de la dérivée seconde est comprise entre -2 et +2 ; dès que ces bornes sont dépassées, il est nécessaire de changer de mode de codage, ce changement de mode étant par exemple matérialisé par un code '11111'.

On obtient ainsi la table de codage suivante (pour laquelle, pour entendu, le choix des bits 0 ou 1 peut être inversé et l'attribution du signe + ou - est arbitraire) :

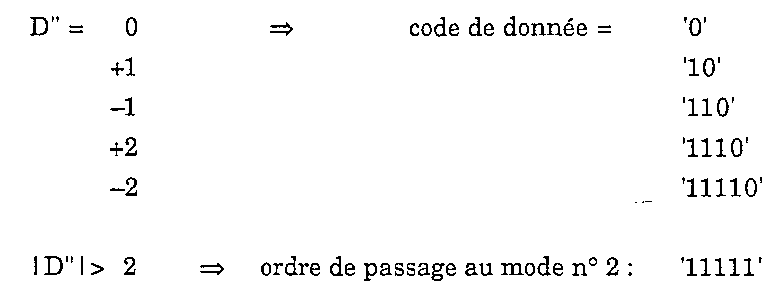

### Mode de codage n°2

Le mode n° 2, mis en oeuvre à l'étape 12, est utilisé dès que la valeur de dérivée seconde dépasse les limites de ± 2, en restant dans les limites de ± 7.

La valeur de dérivée seconde est alors stockée sous la forme d'un mot fixe de 4 bits.

Un code spécifique, par exemple le code -8 ('1000' en binaire) permet de sortir de ce mode, le code étant suivi soit d'un bit à 0 pour repasser en mode n° 1 (si la dérivée seconde se stabilise à des valeurs faibles), soit d'un bit à 1 pour passer au mode n° 3 (si la dérivée seconde dépasse les limites de ± 7).

On obtient le tableau de codage suivant :

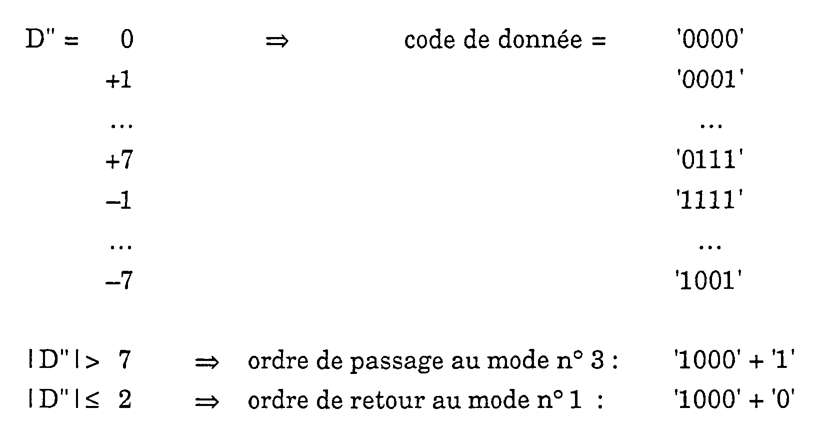

On notera que le codage permis par le mode n° 2 recouvre celui permis par le mode n° 1 (redondance pour les valeurs 0, ± 1 et ± 2), ceci afin d'éviter des basculements incessants de mode, qui préjudicieraient au score global de l'algorithme de compression.

La décision de changement de mode ou de maintien dans le même mode est prise à l'étape 10, décrite plus haut, qui est, comme on l'a indiqué une étape d'évaluation prédictive du score global permettant d'optimiser le nombre de changement de modes par seconde.

### Mode de codage n°3

Le mode n° 3 est mise en oeuvre à l'étape 13.

Pour des valeurs élevées de dérivée seconde (au delà de ± 7), on code non plus la dérivée seconde, mais directement la dérivée première.

Les valeurs de dérivée première sont stockées sur 7 bits soit telles quelles (ce qui correspond à une dynamique de ± 63) soit après transposition par l'intermédiaire d'une table de correspondance logarithmique conservée en mémoire permettant, de manière en elle-même connue, de faire correspondre à une dynamique de sortie de ± 63 une dynamique d'entrée plus grande, par exemple fixé à ± 200. L'erreur introduite par une telle transposition est nulle pour les petites valeurs (la transposition étant par exemple effectuée sans changement pour les valeurs jusqu'à ± 12), puis augmente progressivement avec les valeurs elles-mêmes, tout en restant toujours inférieure à 10 % ; en tout état de cause, puisque l'on code la dérivée première cette erreur ne se cumule pas d'un point à un autre et ne crée donc pas de déformation perceptible sur le tracé ECG.

On obtient le tableau de codage suivant :

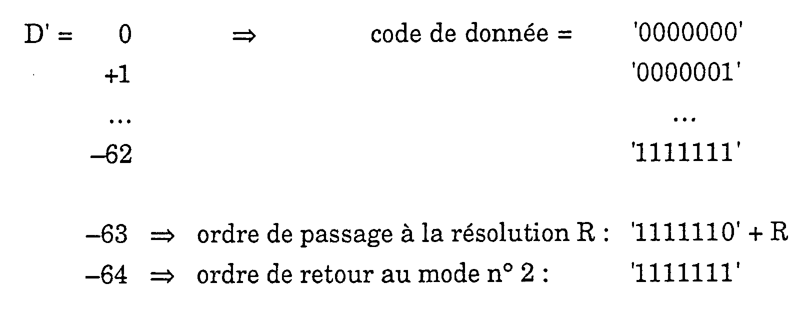

Le code -64 a été réservé pour correspondre à un ordre de retour au mode n° 2, lorsque la dérivée seconde se stabilise à une valeur inférieure à ± 7. On notera que, pour sortir du mode n° 3, on doit avoir enregistré une dérivée première sans erreur. En effet, comme le mode n° 2 (de même que le mode n° 1) n'enregistre que la dérivée seconde, il doit être possible de recalculer lors de la décompression la dérivée première à partir d'une valeur juste pour éviter toute dérive.

Un autre code est réservé. Il s'agit par exemple du code -63, qui sert à introduire un changement de la résolution (diviseur par R de l'étape 7). On notera que le changement de résolution ne s'effectue que lorsque le système est en mode n° 3. Si le module de gestion de la capacité de mémoire (étape 8, décrite plus haut) décide qu'il y a lieu de modifier la résolution, on attend d'être en mode n° 3 pour y procéder ; si le mode n° 3 ne se présente pas dans un laps de temps donné (par exemple dans le cas d'un signal lent après un parasite), l'algorithme peut alors forcer le passage momentané au mode n° 3 pour introduire dans la mémoire le code spécifique de changement de résolution.

### Mémorisation

Les données ainsi codées par l'un quelconque de ces modes de compression sont placées bout à bout dans les octets ou les mots de la mémoire Holter (étape 14 de stockage). On remarquera que, du fait que les données n'ont pas une longueur fixe, une frontière de donnée ne correspond pas nécessairement à une frontière d'octet ou de mot de la mémoire.

### Décompression

La décompression s'effectue en relisant les données de façon séquentielle et en procédant à un codage inverse conforme au système de codage courant (mode n° 1, 2 ou 3). Ainsi, si l'on se trouve en mode n° 1, l'algorithme lit les bits jusqu'à trouver un '0' ou une répétition de cinq '1'. Dans ce dernier cas, il passe au mode n° 2 et lit la séquence de données par fractions de quatre bits successifs, sans difficulté particulière dans la mesure où le codage est de longueur fixe dans ce mode. Dans le cas du mode n° 3, on procède de la même façon, mais par blocs de 7 bits.

Dans les modes n° 1 et n° 2, après avoir ainsi retrouvé la valeur de dérivée seconde, on intègre celle-ci pour obtenir la dérivée première. Dans le mode n° 3, la dérivée première est lue directement (ou après transcodage inverse par la table logarithmique, le cas échéant).

Enfin, l'intégration de la dérivée première donne l'amplitude du signal réel, qui peut être alors affiché sur un écran, stocké dans un mémoire de données, tracé sur un enregistreur à bande, etc., pour permettre son interprétation par le praticien.

Comme on le comprend aisément, la décompression ainsi réalisée ne peut s'effectuer que séquentiellement.

Une première possibilité, la plus simple, consiste à opérer une décompression intégrale du fichier complet, du début jusqu'à la fin.

Malgré la rapidité de la décompression (typiquement 400 à 500 fois le temps réel avec les ordinateurs actuellement utilisés dans ces applications), cette opération préalable peut prendre plusieurs minutes, ce qui peut être gênant lorsque l'on souhaite pouvoir explorer rapidement le signal, notamment lorsque les parties intéressantes de celui-ci se situent vers la fin de l'enregistrement.

Pour permettre un accès plus rapide à un échantillon d'ECG situé à n'importe quel endroit de l'enregistrement, on peut avantageusement, simultanément à la compression, enregistrer périodiquement dans une table d'états (étape 15 sur la figure 1), par exemple à chaque minute, une rubrique donnant l'état instantané du système : mode en cours (n° 1, 2 ou 3), résolution (valeur du facteur R), position du prochain enregistrement dans le fichier (nécessaire lorsqu'une frontière de mot ou d'octet du fichier ne correspond pas à une frontière d'enregistrement) et valeur de la dérivée première.

Lorsque l'on souhaite procéder, au moment du dépouillement de l'enregistrement, à la lecture d'une fraction particulière de cet enregistrement, il suffit de rechercher dans la table l'état des données correspondant à la minute précédant l'instant choisi et effectuer la décompression à partir de cette position dans la mémoire et non plus à partir du tout début de la mémoire, comme dans le cas précédent. On arrive ainsi de façon pratiquement instantanée au point recherché, ce qui permet d'afficher sans délai l'échantillon d'ECG souhaité par le praticien.

Le pas de cette table peut bien entendu être autre qu'un pas à chaque minute, mais doit en pratique rester compris entre quelques secondes et quelques minutes de façon à ne pas occuper un volume trop important dans la mémoire de stockage tout en restant suffisamment performante.

Dans une variante possible, cette table d'états n'est pas constituée en temps réel au moment de l'enregistrement des données dans la mémoire Holter, mais en temps différé lors du premier dépouillement des données, effectué intégralement. Ceci permet de conserver en mémoire dans l'ordinateur du praticien les données sous forme comprimée, avec un gain de place corrélatif.

### Variantes de mise en oeuvre

Le procédé de compression à trois modes (modes n° 1, 2 et 3 décrits plus haut) n'est pas limitatif de l'invention.

Il est possible d'utiliser par exemple un procédé simplifié ne comprenant que deux modes, les modes n° 1 et n° 3 par exemple.

Inversement, il est possible de perfectionner le procédé avec un système à *n* (*n* > 3) modes. En généralisant ainsi le procédé, on peut optimiser la taille des mots inscrits en mémoire en fonction des données incidentes.

Le système garde toujours le principe d'un codage de la dérivée seconde pour les modes n° 1 à *n*-1, et de codage de la dérivée première pour le mode *n*, de niveau le plus élevé. Plus précisément, on calcule sur un nombre de points donnés, c'est-à-dire à un "horizon" qui peut être variable, les valeurs maximales positives et négatives que prendront la dérivée seconde et la dérivée première. En fonction de ces maxima, on sélectionne une taille de mot donnée (par exemple 1 bit pour des zéros successifs, 2 bits pour -1, 0, +1), la valeur négative extrême servant à sortir du mode considéré en cours, cette valeur négative extrême étant immédiatement suivie par un mot sur 3 bits définissant le niveau du mode suivant : n° 1 pour 1 bit, n° 2 pour 2 bits, ... n° 7 pour 7 bits (correspondant à un codage de dérivée première par l'intermédiaire d'une table logarithmique). Le mot zéro est, quant à lui, réservé pour introduire les changements de résolution, qui ont lieu de la même façon et avec les mêmes contraintes que dans le procédé à trois modes décrit plus haut.

## Revendications

1. Un procédé de compression de données physiologiques, **caractérisé par** les étapes successives suivantes :
a) acquisition (1) des données par recueil d'un signal électrique et échantillonnage de ce signal à une fréquence donnée ;
c) détermination (4) de la dérivée première (D') du signal échantillonné (D);
e) détermination (9) de la dérivée seconde (D") de ce même signal échantillonné ;
f) analyse prédictive du signal échantillonné en fonction d'une pluralité de valeurs futures des dérivées première et seconde et sélection (10), pour l'échantillon courant, d'un mode de codage parmi une pluralité de modes de codage (11, 12, 13) prédéfinis commutables dynamiquement, de manière à optimiser le volume global des codes créés pour le codage de ladite pluralité de valeurs futures ; et
g) compression des données par mise en oeuvre du mode de codage (11, 12, 13) choisi à l'étape (f).

2. Le procédé de la revendication 1 dans lequel l'étape (g) comprend :
g1) en cas de changement de mode, la création d'un code de changement de mode, et
g2) la création d'un code de donnée représentatif de la valeur de l'échantillon courant, à partir de la valeur courante correspondante de dérivée première ou de dérivée seconde.

3. Le procédé de la revendication 2, dans lequel, après l'étape (g), il est prévu une étape de :
h) mémorisation séquentielle (14) des codes créés aux étapes (g1) et (g2).

4. Le procédé de la revendication 1, dans lequel, entre l'étape (a) et l'étape (c), il est prévu une étape de :
b) élimination des interférences à la fréquence du secteur par prétraitement (2, 3) des données échantillonnées.

5. Le procédé de la revendication 1, dans lequel, à l'étape (c), la dérivée première est obtenue par détermination de l'incrément entre l'échantillon de signal courant acquis à l'étape (a) et une valeur recalculée correspondant à l'échantillon de signal précédent tel que comprimé à l'étape (g) puis décomprimé par mise en oeuvre d'une étape annexe (6), exécutée de façon concomitante, de codage inverse.

6. Le procédé de la revendication 1, dans lequel il est prévu en outre, entre l'étape (c) et l'étape (e), une étape de :
d) réduction (7), d'un facteur donné (R), de la résolution du signal échantillonné, ce facteur étant déterminé par comparaison entre un débit moyen courant du flux de données mémorisées à l'étape (h) et d'un débit nominal de consigne en augmentant le facteur de réduction lorsque le débit moyen est supérieur au débit nominal, et inversement.

7. Le procédé de la revendication 1, dans lequel ladite pluralité de modes de codage comprend un premier mode de codage (11) dans lequel on mémorise dans un mot de longueur variable la valeur de la dérivée seconde du signal échantillonné, en attribuant une longueur de mot différente pour chaque valeur possible.

8. Le procédé de la revendication 6, dans lequel ledit premier mode de codage est sélectionné lorsque la valeur absolue de la dérivée seconde du signal échantillonné est inférieure à une première limite prédéterminée.

9. Le procédé de la revendication 1, dans lequel ladite pluralité de modes de codage comprend un mode de codage (12), qui est dit second mode de codage, dans lequel on mémorise dans un mot de longueur fixe la valeur de la dérivée seconde du signal échantillonné.

10. Le procédé de la revendication 8, dans lequel ledit second mode de codage est sélectionné lorsque la valeur absolue de la dérivée seconde du signal échantillonné est supérieure à une première limite prédéterminée et inférieure à une deuxième limite prédéterminée.

11. Le procédé de la revendication 1, dans lequel ladite pluralité de modes de codage comprend un mode de codage (13) qui est dit troisième mode de codage, dans lequel on mémorise dans un mot de longueur fixe la valeur de la dérivée première du signal échantillonné.

12. Le procédé de la revendication 1, en ce qui dans ledit troisième mode de codage (13) qui est dit troisième mode de codage, dans lequel on mémorise dans ledit mot de longueur fixe le résultat d'un transcodage logarithmique de la valeur de la dérivée première du signal échantillonné.

13. Le procédé de la revendication 11 ou 12, dans lequel ledit troisième mode de codage est sélectionné lorsque la valeur absolue de la dérivée seconde du signal échantillonné est supérieure à une troisième limite prédéterminée.

14. Le procédé de la revendication 1, comprenant en outre une étape d'enregistrement (15) à intervalles réguliers, dans une table d'états, de la valeur courante de la dérivée première, du mode sélectionné courant et de la position dans l'enregistrement de la donnée mémorisée courante.

15. Un procédé de décompression de données comprimées et mémorisées selon le procédé de l'une des revendications précédentes, **caractérisé par** les étapes successives de :
- lecture séquentielle des données mémorisées,
- détermination du mode de codage de la donnée lue courante, et
- décompression de la donnée par codage inverse, correspondant à ledit mode de codage de la donnée lue courante.

## Patentansprüche

1. Verfahren zur Kompression physiologischer Daten, **gekennzeichnet durch** die folgenden aufeinanderfolgenden Schritte:
a) Erfassung (1) der Daten **durch** Empfang eines elektrischen Signals und Abtasten dieses Signals mit einer gegebenen Frequenz;
c) Bestimmung (4) der ersten Ableitung (D') des abgetasteten Signals (D);
e) Bestimmung (9) der zweiten Ableitung (D") des gleichen abgetasteten Signals;
f) Prädiktive Analyse des abgetasteten Signals abhängig von einer Vielzahl von zukünftigen Werten der ersten und zweiten Ableitung, und Auswahl (10), für den aktuellen Abtastwert, eines Kodierungsmodus unter einer Vielzahl von vordefinierten, dynamisch vertauschbaren Kodierungsmodi (11, 12, 13), um das Gesamtvolumen der **durch** die Kodierung der genannten Vielzahl von zukünftigen Werten erzeugte Volumen zu optimieren; und
g) Kompression der Daten **durch** Verwirklichung des in Schritt (f) ausgewählten Kodierungsmodus (11, 12, 13).

2. Verfahren nach Anspruch 1, in welchem Schritt (g) umfasst:
g1) im Fall der Änderung des Modus die Erzeugung eines Modus-Änderungscodes, und
g2) die Erzeugung eines Datencodes, der repräsentativ für den aktuellen Abtastwert ist, ausgehend von dem entsprechenden aktuellen Wert der ersten Ableitung oder der zweiten Ableitung.

3. Verfahren nach Anspruch 2, in welchem, nach Schritt (g), ein Schritt vorgesehen ist, der:
h) sequentiellen Speicherung (14) der in den Schritten (g1) und (g2) erzeugten Codes.

4. Verfahren nach Anspruch 1, in welchem, zwischen Schritt (a) und (c) ein Schritt vorgesehen ist, der:
b) Beseitigung der Interferenzen mit der Netzfrequenz durch Vorbehandlung (2, 3) der abgetasteten Daten.

5. Verfahren nach Anspruch 1, in welchem, in Schritt (c), die erste Ableitung erhalten wird durch Bestimmung des Inkrements zwischen dem aktuellen Abtastwert des Signals, der in Schritt (a) erfasst wurde, und einem wiederberechneten Wert, der dem vorhergehenden Abtastwert des Signals entspricht, wie etwa in Schritt (g) komprimiert, dann dekomprimiert durch Verwirklichung eines in begleitender Weise ausgeführten Zusatzschritts (6) der umgekehrten Kodierung.

6. Verfahren nach Anspruch 1, in welchem ferner zwischen Schritt (c) und Schritt (e) ein Schritt vorgesehen ist, der:
d) Reduktion (7) eines gegebenen Faktors (R) der Auflösung des abgetasteten Signals, wobei dieser Faktor bestimmt wird durch Vergleich zwischen einem aktuellen mittleren Durchsatz des Flusses der in Schritt (h) gespeicherten Daten und eines nominalen Soll-Durchsatzes, in welchem der Faktor der Reduktion erhöht wird, wenn der mittlere Durchsatz größer als der nominale Durchsatz ist, und umgekehrt.

7. Verfahren nach Anspruch 1, in welchem die Mehrzahl von Kodierungsmodi einen ersten Kodierungsmodus (11) umfasst, in welchem in einem Wort variabler Länge der Wert der zweiten Ableitung des abgetasteten Signals gespeichert wird, indem für jeden möglichen Wert eine unterschiedliche Wortlänge zugewiesen wird.

8. Verfahren nach Anspruch 6, in welchem der erste Kodierungsmodus ausgewählt wird, wenn der absolute Wert der zweiten Ableitung des abgetasteten Signals kleiner als ein erster vorherbestimmter Grenzwert ist.

9. Verfahren nach Anspruch 1, in welchem die Vielzahl von Kodierungsmodi einen Kodierungsmodus (12) umfasst, der zweiter Kodierungsmodus genannt wird, in welchem in einem Wort fester Länge der Wert der zweiten Ableitung des abgetasteten Signals gespeichert wird.

10. Verfahren nach Anspruch 8, in welchem der zweite Kodierungsmodus ausgewählt wird, wenn der absolute Wert der zweiten Ableitung des abgetasteten Signals größer als ein erster vorherbestimmter Grenzwert und kleiner als ein zweiter vorherbestimmter Grenzwert ist.

11. Verfahren nach Anspruch 1, in welchem die Vielzahl von Kodierungsmodi einen Kodierungsmodus (13) umfasst, der dritter Kodierungsmodus genannt wird, in welchem in einem Wort fester Länge der Wert der ersten Ableitung des abgetasteten Signals gespeichert wird.

12. Verfahren nach Anspruch 1, **dadurch gekennzeichnet, dass** in dem genannten dritten Kodierungsmodus (13), in dem genannten Wort fester Länge das Ergebnis einer logarithmischen Transkodierung des Werts der ersten Ableitung des abgetasteten Signals gespeichert wird.

13. Verfahren nach Anspruch 11 oder 12, in welchem der dritte Kodierungsmodus ausgewählt wird, wenn der absolute Wert der zweiten Ableitung des abgetasteten Signals größer als ein dritter vorherbestimmter Grenzwert ist.

14. Verfahren nach Anspruch 1, ferner umfassend einen Schritt der Aufzeichnung (15) in regelmäßigen Intervallen, in einer Tabelle von Zuständen, des aktuellen Werts der ersten Ableitung, des aktuellen ausgewählten Modus und der Position in der Aufzeichnung des aktuellen gespeicherten Datums.

15. Verfahren zur Dekomprimierung von Daten, die gemäß dem Verfahren eines der vorhergehenden Ansprüche komprimiert und gespeichert wurden, **gekennzeichnet durch** die aufeinanderfolgenden Schritte:
- des sequentiellen Lesens der gespeicherten Daten,
- der Bestimmung des Kodierungsmodus des aktuellen gelesenen Datums, und
- der Dekomprimierung des Datums **durch** umgekehrte Kodierung, entsprechend dem Kodierungsmodus des aktuellen gelesenen Datums.

## Claims

1. A method for the compression of physiological data, **characterised by** the following successive steps:
a) acquisition (1) of data by sensing an electrical signal and sampling said signal at a given frequency;
c) determination (4) of the first derivative (D') of the sampled signal (D);
e) determination (9) of the second derivative (D") of the same sampled signal (D);
f) predictive analysis of the sampled signal as a function of a plurality of future values of the first and second derivatives and selection (10), for the current sampling, of a coding mode among a plurality of predefined, dynamically switchable coding modes (11, 12, 13) so as to optimize the global volume of the codes created for coding said plurality of future values; and
g) compression of the data by implementation of the coding mode (11, 12, 13) selected at step (f).

2. The method of claim 1, wherein step (g) includes:
g1) in case of a change of mode, the creation of a change of mode code, and
g2) the creation of a data code representative of the value of the current sample, from the corresponding current value of the first or second derivatives.

3. The method of claim 2, wherein there is provided after step (g) a step of:
h) sequential storing (14) of the codes created at steps (g1) and (g2).

4. The method of claim 1, wherein there is provided between step (a) and step (c) a step of:
b) elimination of power-line frequency interferences by pre-processing (2, 3) of the sampled data.

5. The method of claim 1, wherein at step (c) the first derivative is obtained by determining the increment between the current signal sample acquired at step (a) and a recalculated value corresponding to the previous signal sample as compressed at step (g) then decompressed by implementation of an ancillary, simultaneously performed, reverse-coding step (6).

6. The method of claim 1, wherein there is provided between step (c) and step (e) a step of:
d) reduction (7), by a given factor (R), of the resolution of the sampled signal, said factor being determined by a comparison between a current average rate of the flow of the data stored at step (h) and a set nominal rate by increasing the reduction factor when the average rate is greater than the nominal rate, and conversely.

7. The method of claim 1, wherein said plurality of coding modes includes a first coding mode (11) in which the value of the second derivative of the sampled signal is stored in a variable-length word, by giving a different word length to every possible value.

8. The method of claim 7, wherein said first coding mode is selected when the absolute value of the second derivative of the sampled signal is less than a first predetermined limit.

9. The method of claim 1, wherein said plurality of coding modes includes a coding mode (11), which is called a second coding mode, in which the value of the second derivative of the sampled signal is stored in a fixed-length word.

10. The method of claim 9, wherein said second coding mode is selected when the absolute value of the second derivative of the sampled signal is greater than a first predetermined limit and less than a second predetermined limit.

11. The method of claim 1, wherein said plurality of coding modes includes a coding mode (13), which is called a third coding mode, in which the value of the first derivative of the sampled signal is stored in a fixed-length word.

12. The method of claim 1, wherein in said third coding mode (13) the result of a logarithmic conversion of the value of the first derivative of the sampled signal is stored in said fixed-length word.

13. The method of claim 11 or 12, wherein said third coding mode is selected when the absolute value of the second derivative of the sampled signal is greater than a third predetermined limit.

14. The method of claim 1, further comprising a step of recording (15) at regular intervals, in a status table, the current value of the first derivative, the current coding mode, and the position in the record of the current recorded data.

15. A method of decompression of data compressed and stored according to the method of any of the preceding claims, **characterised by** the successive steps of:
- sequential reading of the stored data;
- determination of the coding mode of the current data read; and
- decompression of the data by reverse coding, corresponding to said coding mode of the current data read.
